# EUROPÄISCHE PATENTSCHRIFT

(11) **EP 2 362 756 B1**
(45) Veröffentlichungstag und Bekanntmachung des Hinweises auf die Patenterteilung: **29.04.2015**
(21) Anmeldenummer: 09778185.0
(22) Anmeldetag: 28.08.2009
(51) Int. Cl.: A61B 18/00, A61B 18/12

(54) **VORRICHTUNG ZUR DEVITALISIERUNG VON BIOLOGISCHEM GEWEBE**
DEVICE FOR DEVITALIZING BIOLOGICAL TISSUE
DISPOSITIF DE DÉVITALISATION DE TISSU BIOLOGIQUE

(30) Priorität: 07.10.2008 DE 102008050635
(43) Veröffentlichungstag der Anmeldung: 07.09.2011
(73) Patentinhaber: ERBE Elektromedizin GmbH, 72072 Tübingen (DE)
(72) Erfinder: EISELE, Florian, 79108 Freiburg (DE); VOIGTLÄNDER, Matthias, 72810 Gomaringen (DE)
(74) Vertreter: Bohnenberger, Johannes
(86) Internationale Anmeldenummer: PCT/EP2009/006255
(87) Internationale Veröffentlichungsnummer: WO 2010/040431

(56) Entgegenhaltungen:
- WO-A1-2008/077317
- US-A- 4 658 819
- US-A- 5 720 744
- US-A1- 2001 014 819
- US-B1- 6 692 489

## Beschreibung

Die Erfindung betrifft eine Vorrichtung zur Devitalisierung von biologischem Gewebe gemäß dem Oberbegriff des Anspruchs 1.

Es sind elektrochirurgische Geräte, insbesondere Sonden zur Devitalisierung von Gewebe bekannt (Ablationssonden), die einen Sondenkörper mit mindestens einer Elektrode zum Anlegen einer HF-Spannung und eine Kühleinrichtung umfassen. Die HF-Spannung wird über einen HF-Generator (Hochfrequenzgenerator) erzeugt.

Bei der Hochfrequenzchirurgie wird Wechselstrom mit hoher Frequenz durch den menschlichen Körper geleitet, um Gewebe gezielt zu schädigen. Ein Anwendungsgebiet der Hochfrequenzchirurgie ist die Devitalisierung von Tumorgewebe. Hochfrequenzchirurgie macht sich den thermischen Effekt der Erwärmung zu Nutze, durch den die Devitalisierung erzielt wird.

Man unterscheidet zwischen einer bipolaren und einer monopolaren Applikation des HF-Stroms. Bei einer monopolaren Anwendung umfasst das Instrument des elektrochirurgischen Geräts lediglich eine Elektrode, während eine zweite, Neutralelektrode unmittelbar an dem Patienten angebracht ist. Der Strom fließt umgekehrt proportional zu den Widerstandsverhältnissen im Gewebe von der Elektrode des Instruments zu der Neutralelektrode. In unmittelbarer Nähe der Elektrode des Instruments ist die Stromdichte derart hoch, dass der beschriebene thermische Effekt auftritt. Mit zunehmendem Abstand zu dieser Elektrode nimmt die Stromdichte quadratisch ab. Somit ist die devitalisierende Wirkung des HF-Stroms räumlich begrenzt.

Bei einer bipolaren Applikation umfasst das Instrument zwei Elektroden. Beispielsweise kann eine Sondenspitze als erste Elektrode ausgebildet sein, während ein proximaler Abschnitt der Sonde als zweite Elektrode dient. Die HF-Spannung liegt zwischen den beiden gegeneinander isolierten Elektroden an. Der Stromkreis wird über das dazwischen liegende Gewebe geschlossen. Es ergibt sich ein Stromverteilungsfeld, das sich um die Sonde konzentriert.

Es ist offensichtlich, dass sich unabhängig von der Applikationsart des HF-Stroms (monopolar oder bipolar) eine hohe Felddichte in unmittelbarer Nachbarschaft zu den Instrumenten einstellt. Diese Felddichte kann zur Dehydration, bis hin zur Karbonisation des umliegenden Gewebes führen. Dieser Effekt ist zumindest bei der Devitalisierung von Tumoren unerwünscht, da dehydriertes oder karbonisiertes Gewebe eine stark isolierende Wirkung hat und die Behandlung in tieferen Gewebeschichten hemmt. Des Weiteren kann der Körper karbonisiertes Gewebe nicht ohne Weiteres abbauen.

Deshalb wird eine Kühleinrichtung eingesetzt, um unmittelbar benachbartes Gewebe zu kühlen und so eine Dehydration und/oder Karbonisation des anliegenden Gewebes zu vermeiden.

Solche Vorrichtungen sind zum Beispiel aus WO 2008/077317 A1 oder US 2001/0014819 A1 bekannt.

Je nach Größe des Tumors kann der erzielte Devitalisierungseffekt mit einem Instrument in Volumen und/oder Geschwindigkeit nicht ausreichend sein, um den Tumor, vollständig unter Berücksichtigung eines onkologischen Sicherheitsrandes zu devitalisieren. In diesem Fall werden Cluster-Elektroden und/oder eine Vielzahl von Ablationssonden, die als Cluster-Elektroden wirken, eingesetzt. Cluster-Elektroden können aus zwei bis vier geometrisch zueinander angeordneten Einzelelektroden bestehen, welche jeweils mit einer HF-Spannung versorgt werden. Bei deren Einsatz werden sowohl monopolare als auch bipolare Ausführungen verwendet, welche von einem Generator versorgt werden und im Parallel- oder Multiplexbetrieb betrieben werden.

Bei mehreren mit konstanter Leistung parallel betriebenen gekühlten Applikationssonden an einem Generator kann es beim Start der Applikation zu unsymmetrischen Stromverteilungen zwischen den Elektroden aufgrund unterschiedlicher Ausgangssituationen (Impedanz-, Wärmekapazitäts-, Wärmeleitungsverhältnisse) kommen.

Es ist zwar bekannt, elektrochirurgische Instrumente lastabhängig derart zu regeln, dass die besagte Dehydration oder Karbonisierung nicht auftritt (vgl. DE 42 33 467 A1), jedoch lassen sich diese Ansätze nicht ohne Weiteres bei der Verwendung von Cluster-Elektroden verwenden.

So kann es beispielsweise dazu kommen, dass ein erstes Elektrodenpaar einen geringen Stromeintrag (Es wird wenig HF-Energie in das Gewebe eingebracht.) hat, während ein zweites Elektrodenpaar über einen wesentlich höheren Stromeintrag verfügt. Bei konstanter Kühlung des ersten Elektrodenpaars steigt aufgrund der sinkenden Temperatur der Übergangswiderstand, wodurch wiederum der Stromeintrag an diesem ersten Elektrodenpaar verringert wird. Dies kann in einem ungünstigen Fall dazu führen, dass der Stromeintrag an dem zweiten Elektrodenpaar weiter steigt. An dem zweiten Elektrodenpaar entsteht ein Missverhältnis zwischen der aufgebrachten Kühlleistung und der HF-Leistung. Es kommt zu einer Austrocknung des Gewebes, wodurch sich der Übergangswiderstand auch an der zweiten Elektrode erhöht.

Es kann durch Messungen des Übergangswiderstands an den einzelnen Elektroden festgestellt werden, ob der Devitalisierungsvorgang oder Koagulationsvorgang als abgeschlossen einzustufen ist. Bei dem beschriebenen Anstieg des Übergangswiderstandes an dem ersten Elektrodenpaar (wegen zu starker Kühlung) und dem zweiten Elektrodenpaar (wegen zu hoher HF-Leistung), kann es zu einer Fehleinschätzung kommen, bei der der Koagulationsvorgang fälschlicherweise als abgeschlossen eingestuft wird. Es ist offensichtlich, dass die fehlende oder zu geringe Applikation eines HF-Stroms an der ersten Elektrode das Gesamtergebnis des Eingriffs negativ beeinflusst. Des Weiteren kann die kurze Applikation des HF-Stroms mit hoher Spannung an dem zweiten Elektrodenpaar dazu führen, dass von den Elektroden weit entferntes Gewebe nicht devitalisiert wird.

Wird der Devitalisierungsvorgang aufgrund des Anstiegs des Übergangswiderstands an dem ersten Elektrodenpaar und dem zweiten Elektrodenpaar nicht unterbrochen, so tritt ein selbstregelnder Effekt auf. Da der Übergangswiderstand an dem zweiten Elektrodenpaar wesentlich stärker ansteigt als an dem ersten Elektrodenpaar, verschiebt sich die HF-Leistungsverteilung zu Gunsten des ersten Elektrodenpaars. Soweit an dem zweiten Elektrodenpaar "nur" eine Dehydration vorliegt, kann es zu einer Rückdiffusion von Gewebeflüssigkeit kommen. Der Übergangswiderstand an dem zweiten Elektrodenpaar sinkt, während der an dem ersten Elektrodenpaar möglicherweise aufgrund einer Austrocknung des Gewebes steigt. Das System neigt also dazu, zwischen den einzelnen Zuständen hin und her zu schwingen, wobei die HF-Leistung an den einzelnen Elektrodenpaaren ansteigt und wieder abfällt. Voraussetzung hierfür ist es, dass keine irreversiblen Effekte an dem Übergangsgewebe auftreten.

Das System neigt jedoch zum Überschwingen, insbesondere bei größeren Abständen zwischen den Elektroden-Clustern, da hier die Abgabe von zuviel Kühlenergie an der einen Sonde nicht durch die Wärmediffusion von der anderen Sonde abgeschwächt wird.

Beim Mehrfachbetrieb der Sonden in den Clustern oder in Arrays wird ein übermäßiger Impedanzanstieg dadurch verhindert, dass die Ablationssonden bzw. Sonden oder Sondenpaare nacheinander mit einem bestimmten Algorithmus betrieben werden. Dies hat jedoch den Nachteil, dass keine effiziente Devitalisierung durch einen kontinuierlichen Energieeintrag in das Gewebe möglich ist. Selbst bei der Verwendung von nur einer Ablationssonde reichen herkömmliche Regelalgorithmen nicht aus, um eine zu starke Kühlung oder Überhitzung des unmittelbar benachbarten Gewebes zu verhindern. Beispielsweise ist es bekannt, eine Regelung anhand der Steilheit des Stromabfalls (dI/dt) bzw. des Impedanzanstiegs (dZ/dt) vorzunehmen. Eine derartige Regelung ist aber unzureichend.

Daher ist es Aufgabe der vorliegenden Erfindung, die Regelung gekühlter Ablationssonden sowohl im Einzelbetrieb als auch im Clusterbetrieb zu verbessern. Insbesondere soll eine Vorrichtung zur Devitalisierung von biologischem Gewebe bereitgestellt werden, die eine zuverlässige und für den Patienten schonende Devitalisierung von vorgegebenen Gewebeabschnitten ermöglicht. Des Weiteren soll ein entsprechendes Verfahren angegeben werden.

Diese Aufgabe wird durch eine Vorrichtung gemäß dem vorliegenden Anspruch 1 gelöst.

Insbesondere wird die Aufgabe bei einer Vorrichtung zur Devitalisierung von biologischem Gewebe, umfassend:
mindestens eine Ablationssonde, die derart ausgebildete und angeordnete Kühleinrichtungen aufweist, dass durch deren Kühlleistung Gewebebereiche nahe der Ablationssonde kühlbar sind, und die derart angeordnete und ausgebildete Elektrodeneinrichtungen aufweist, dass ein von einem HF-Generator erzeugter HF-Behandlungsstrom in das Gewebe einleitbar ist,
eine Regeleinrichtung gelöst, die derart ausgebildet und mit den Kühleinrichtungen sowie mit dem HF-Generator verbunden ist, dass zu Beginn der Devitalisierung bei einer niedrigen, ersten Temperatur und am Ende der Devitalisierung bei einer erhöhten, zweiten Temperatur gearbeitet wird.

Der Kern der Erfindung beruht auf der Erkenntnis, dass die Gewebeveränderung aufgrund einer zu starken Kühlung im Wesentlichen reversibel ist, während die Austrocknung und/oder Karbonisierung des Gewebes aufgrund einer zu starken Erhitzung sich nicht oder nur sehr langsam regeneriert. Daher ist es sinnvoll, rechtzeitig, d.h. von Anfang an, mittels der Kühleinrichtung regulierend einzugreifen. Erfindungsgemäß wird also am Beginn der Devitalisierung wesentlich stärker gekühlt, während gegen Ende des Verfahrens die Temperatur erhöht wird. Die Temperatur kann entweder über das Regeln der Kühlleistung oder über eine Regelung des Verhältnisses zwischen HF-Leistung und Kühlleistung erfolgen. Bei der ersten Temperatur wird ein Stromeintrag in die weiter entfernten Gewebegebiete ermöglicht und somit eine ausreichende Ausdehnung der Koagulationszone sichergestellt. Nach dem Erreichen des gewünschten Koagulationsradius wird bei einem Anliegen der zweiten Temperatur das der Ablationssonde benachbarte Gewebe devitalisiert.

Die Regeleinrichtung kann derart ausgebildet sein, dass die Temperatur so eingestellt wird, dass keine Vereisung des Gewebes auftritt. Es ist vorteilhaft, die Vorrichtung derart zu betreiben, dass die erste Temperatur so gewählt wird, dass diese nahe dem Gefrierpunkt des zu behandelnden Gewebes, insbesondere zwischen 1 und 8°C liegt. Jedoch sollte die Regeleinrichtung derart ausgebildet sein, dass ein Vereisen des Gewebes vermieden wird. Mit einer Vereisung des Gewebes steigt der spezifische Widerstand, weswegen ein geringer Stromeintrag in das Gewebe auftreten kann.

Die Regeleinrichtung kann zum Einstellen der ersten Temperatur durch Vorgabe eines ersten Behandlungsstroms und zum Einstellen der zweiten Temperatur durch Vorgabe einer ersten Behandlungsspannung und jeweils einer entsprechenden Einstellung der Kühlleistung ausgebildet sein. Da die Widerstandsänderung in einem großen Bereich nahe der ersten Temperatur negativ ist, kann bei einem Betrieb mit einem im Wesentlichen konstanten ersten Behandlungsstrom eine stabile Regulierung des Koagulationsvorgangs gewährleistet werden. Da die Widerstandsänderungen in einem großen Bereich nahe der zweiten Temperatur positiv ist, kann in diesem zweiten Betriebsmodus eine stabile Einstellung des Koagulationsvorgangs mit einer im Wesentlichen konstanten Behandlungsspannung erzielt werden.

Die Regeleinrichtung kann eine konstante Stromquelle zur Einstellung des ersten Behandlungsstroms umfassen.

Die Regeleinrichtung kann eine konstante Spannungsquelle zur Einstellung der ersten Behandlungsspannung umfassen.

Die Regeleinrichtung kann derart eingestellt sein, dass ein gleitender Übergang von einer Stromregelung zu einer Spannungsregelung durchgeführt wird.

Die Regeleinrichtung kann zur Feststellung der ersten und/oder der zweiten Temperatur eine Impedanzmesseinrichtung zur Messung einer Impedanz zwischen der Elektrodeneinrichtung und dem umgebenden Gewebe aufweisen. Beispielsweise kann die Impedanz zwischen der Neutralelektrode und der Applikationselektrode gemessen werden. Alternativ kann die Impedanz zwischen Elektrodenpaaren gemessen werden, die zur Applikation des HF-Stroms mit einer Spannung versorgt werden. Auch können einzelne Elektroden derart mehrteilig aufgebaut sein, dass eine Messung zwischen den Elektrodenteilen erfolgt. Die Impedanzmessung kann vorteilhaft verarbeitet werden, um Rückschlüsse über die an der Ablationssonde vorliegenden Bedingungen, insbesondere die vorliegende Temperatur, zu ziehen.

Die Vorrichtung kann eine Vielzahl von Ablationssonden und Regeleinrichtungen umfassen, wobei eine übergeordnete Steuer-/Regeleinrichtung vorgesehen ist, die derart ausgebildet ist, dass die jeweiligen Ablationssonden mit dazugehörigen Regeleinrichtungen gemeinsam steuerbar sind. Besonders beim Betreiben einer Vielzahl von Ablationssonden ist es vorteilhaft, eine Dampfbildung und Austrocknung am Beginn des Devitalisierungs- oder Koagulationsvorgangs zu vermeiden, indem die einzelnen Ablationssonden mit der ersten niedrigeren Temperatur betrieben werden.

Beschrieben wird ebenfalls ein Verfahren zum Regeln einer Vorrichtung zur Devitalisierung von biologischem Gewebe, wobei die Vorrichtung mindestens eine Ablationssonde umfasst, die derart ausgebildete und angeordnete Kühleinrichtungen aufweist, dass durch deren Kühlleistung Gewebebereiche nahe der Ablationssonde kühlbar sind und die derart angeordnete und ausgebildete Elektrodeneinrichtungen aufweist, dass ein Behandlungsstrom in das Gewebe einleitbar ist, wobei zu Beginn der Devitalisierung bei einer niedrigen, ersten Temperatur am Ende der Devitalisierung bei einer höheren, zweiten Temperatur gearbeitet wird.

Die Vorteile, die durch das Verfahren erzielt werden, entsprechen im Wesentlichen denen der bereits beschriebenen Vorrichtung.

Nachfolgend wird die Erfindung anhand von einigen Ausführungsbeispielen beschrieben, die anhand der Abbildungen näher erläutert werden. Hierbei zeigen:
- - Fig. 1: die wesentlichen Komponenten der erfindungsgemäßen Vorrichtung zur Devitalisierung von biologischem Gewebe;
- - Fig. 2: mehrere Ablationssonden mit der erfindungsgemäßen Regel-/Steuereinrichtung; und
- - Fig. 3: ein Diagramm zur Verdeutlichung der Abhängigkeit des spezifischen Widerstands von biologischem Gewebe zur Temperatur.

In der nachfolgenden Beschreibung werden für gleiche und gleich wirkende Teile dieselben Bezugsziffern verwendet.

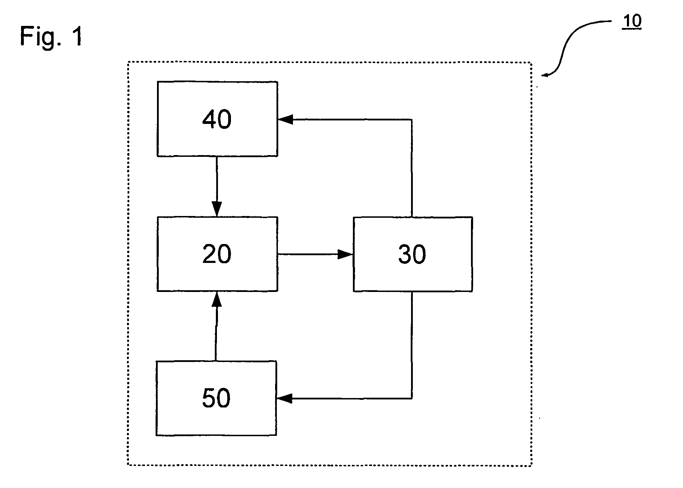

Ein erstes Ausführungsbeispiel der erfindungsgemäßen Vorrichtung 10 zur Devitalisierung von biologischem Gewebe umfasst eine Ablationssonde 20 und eine Regel-/Steuereinrichtung 30 (vgl. Fig. 1). Zur Versorgung der Ablationssonde 20 mit einer HF-Spannung ist diese an einen HF-Generator 50 angeschlossen. Des Weiteren gibt es eine fluide Verbindung zwischen einer Kältemittelquelle 40, die Kältemittel zur Kühlung der Ablationssonde 20 bereitstellt. Die Regel-/Steuereinrichtung 30 steuert bzw. regelt die Kältemittelquelle 40 derart, dass die Ablationssonde mit einer vorgegebenen Kühlleistung P_{K} gekühlt wird. Des Weiteren steuert bzw. regelt die Regel-/Steuereinrichtung 30 den HF-Generator 50 derart, dass die Ablationssonde 20 eine vorgegebene HF-Leistung P_{HF} an das Gewebe abgibt.

Um die HF-Leistung P_{HF} und die Kühlleistung P_{K} geeignet zu regeln, empfängt die Regel-/Steuereinrichtung 30 Messsignale von der Ablationssonde 20.

In dem gezeigten Ausführungsbeispiel soll eine spezifische Leitfähigkeit oder ein spezifischer Widerstand 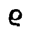 des Gewebes gemessen werden.

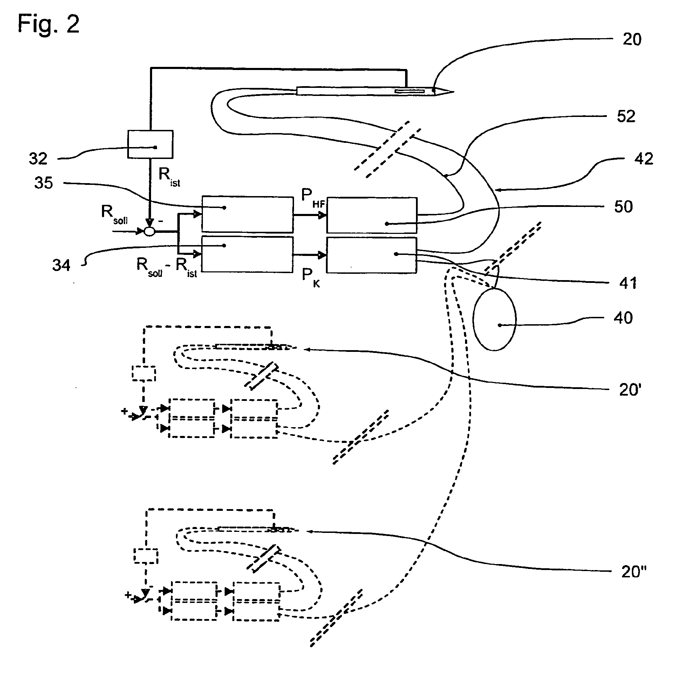

Wie in der Fig. 2 schematisch angedeutet, umfasst die Vorrichtung 10 hierfür eine Messeinrichtung 32, die eine vorliegende Ist-Impedanz Rᵢₛₜ erfasst. Diese Ist-Impedanz Rᵢₛₜ kann zwischen einem oder mehreren Elektrodenpaaren gemessen werden. Denkbar ist es auch, eine Elektrode zwei oder mehrteilig aufzubauen und den Widerstand zwischen den einzelnen Teilen der Elektrode zu messen.

Die spezifische Leitfähigkeit des Gewebes gibt Aufschluss über die Temperatur des Gewebes und dessen Zustand (z.B. vereist, ausgetrocknet oder karbonisiert).

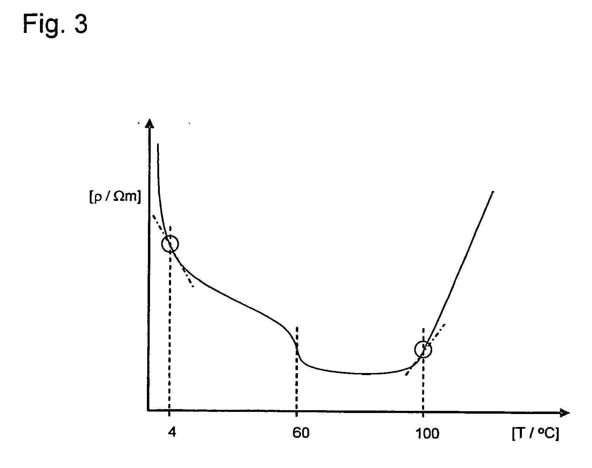

Die spezifische Leitfähigkeit des Gewebes wird im Verlauf der Behandlung durch die eingebrachte oder entzogene Energie, also durch HF-Leistung P_{HF} und Kühlleistung P_{K} beeinflusst. So sinkt die spezifische Leitfähigkeit (spezifische Leitfähigkeit = 1/ 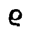 ), wenn sich die Gewebetemperatur dem Gefrierpunkt annähert (vgl. hierzu Fig. 3). Andererseits kommt es bei hohen Temperaturen, insbesondere größer gleich 100°C zu einer Dampfbildung und Austrocknung des Gewebes. Somit sinkt die spezifische Leitfähigkeit deutlich, wenn das Gewebe eine Temperatur größer gleich 100° hat.

Die Regel-/Steuereinrichtung 30 nutzt die Stellgrößen HF-Leistung P_{HF} und Kühlleistung P_{K} um das Gewebe bei einer vorgegebenen Temperatur zu koagulieren. Insbesondere ist es Teil der Offenbarung, im zu behandelnden Gewebe einen vorgegebenen Temperaturgradienten zu erzielen.

Abstrakt betrachtet, wird eine Soll-Impedanz Rₛₒₗₗ mit der gemessenen Ist-Impedanz Rᵢₛₜ verglichen und die Stellgrößen gemäß dem erzielten Ergebnis eingestellt. Zur Regelung der HF-Leistung P_{HF} und zur Regelung der Kühlleistung P_{K} umfasst die Regel-/Steuervorrichtung 30 jeweils einen Kühlleistungsregler 34 und einen HF-Leistungsregler 35.

Der HF-Leistungsregler 35 kann beispielsweise den HF-Generator 50 steuern, der über eine Leitung 52 die Ablationssonde 20 mit dem HF-Strom versorgt. Der Kühlleistungsregler 34 kann über die Regelung eines Ventils 41 die Kältemittelzufuhr aus der Kältemittelquelle 40 regeln und somit die Kühlleistung P_{K} einstellen.

Die Vorrichtung 10 zur Devitalisierung von Gewebe umfasst mindestens zwei Betriebszustände. In einem ersten Betriebszustand wird die Ablationssonde 20 an einem ersten Arbeitspunkt AP1 betrieben und in einem zweiten Betriebszustand an einem zweiten Arbeitspunkt AP2. Der erste Arbeitspunkt AP1 zeichnet sich durch eine erste, niedrige Temperatur, insbesondere im Intervall zwischen 2° und 10°C und der zweite Arbeitspunkt AP2 durch eine deutlich höhere, zweite Temperatur, insbesondere im Intervall von 80° bis 110°C aus. Die angegebenen Temperaturangaben beziehen sich insbesondere auf das der Ablationssonde 20 unmittelbar benachbarte Gewebe und/oder auf die Temperatur ah der Außenhüllen der Ablationssonde 20.

Im ersten Arbeitspunkt AP1 wird durch das Kühlen des Gewebes um die Sondenoberfläche herum ein Stromeintrag in die weiter entfernten Gewebegebiete ermöglicht. Der thermische Effekt zur Devitalisierung von Gewebe tritt also in Bereichen auf, die von der applizierenden Ablationssonde 20 relativ weit entfernt sind. Bei einer Abweichung um den ersten Arbeitspunkt AP1 tritt keine irreversible Veränderung des Gewebes durch Austrocknung auf (vgl. Denaturierung bei dem zweiten Arbeitspunkt AP2). Ein Vereisen des benachbarten Gewebes wäre eine reversible Veränderung, die verhältnismäßig unproblematisch ist. Insbesondere kann das Gewebe innerhalb weniger Sekunden wieder aufgetaut werden. Es ergibt sich also eine Temperaturverteilung, wobei in unmittelbarer näher eine relativ niedrige Temperatur vorliegt (z. B. die erste Temperatur) und die Temperatur mit zunehmender Entfernung bis zu einem Maximalwert zunimmt. Nach dem erreichen dieses Maximalwerts nimmt die Temperatur wieder bis auf Körpertemperatur ab.

Nach dem Erreichen des gewünschten Koagulationsradius nimmt die Vorrichtung 10 den zweiten Betriebszustand ein und regelt die Stellgrößen derart, dass der zweite Arbeitspunkt AP2 erreicht wird. Denkbar ist es auch, einen kontinuierlichen Übergang von der Regelung im ersten Arbeitspunkt zu der Regelung im zweiten Arbeitspunkt AP2 vorzunehmen. Somit kann der Koagulationsradius schrittweise verringert werden. Da die spezifische Widerstandsänderung ( 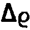 /ΔT) in einem großen Intervall um den ersten Arbeitspunkt AP1 negativ ist, kann bei Betrieb mit Konstantstrom ein stabiler Arbeitspunkt realisiert werden.

Andererseits ist die spezifische Widerstandsänderung ( 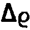 /ΔT) in einem großen Intervall um den zweiten Arbeitspunkt AP2 positiv, weswegen bei Betrieb mit Konstantspannung ein stabiler zweiter Arbeitspunkt realisiert werden kann.

Wendet man sich der Fig. 3 zu, die den spezifischen Widerstand von biologischem Gewebe in Abhängigkeit von der vorliegenden Temperatur zeigt (die Abszisse entspricht der Temperatur in °C, die Ordinate dem spezifischen Widerstand in Ohmmetern), so zeigt sich, dass die dort gewählten Arbeitspunkte AP1 und AP2 eine entsprechende Widerstandsänderung aufweisen. Es zeigt sich weiterhin, dass ein erster Arbeitspunkt AP1 bei ca. 4° C besonders vorteilhaft ist, da bei einer tieferen Temperatur der spezifische Widerstand drastisch ansteigt. Dieser Arbeitspunkt AP1 lässt sich also einfach detektieren.

Des Weiteren zeigt es sich, dass der zweite Arbeitspunkt AP2 mit einer Temperatur von ca. 100° C ebenfalls sehr charakteristisch ist, da bei höheren Temperaturen der spezifische Widerstand ebenfalls drastisch ansteigt. Somit ist das Auffinden dieser charakteristischen Arbeitspunkte AP1 und AP2 und deren Einhalten relativ einfach realisierbar.

In einem weiteren Ausführungsbeispiel werden, wie in Fig. 2 gezeigt, eine Vielzahl von Ablationssonden 20, 20', 20" gleichzeitig mit einer Vielzahl von Regel-/Steuereinrichtungen 30 betrieben, um ein großes Tumorgebiet zu devitalisieren. Soweit die einzelnen Ablationssonden 20, 20', 20" ähnlich geregelt bzw. gesteuert werden wie dies vorab beschrieben wurde, kommt es zu einer vorteilhaften Devitalisierung des Gewebes. Insbesondere kann eine vollständige und für den Patienten schonende Devitalisierung von großen Gewebeabschnitten erzielt werden.

### Bezugszeichenliste

10 Vorrichtung zur Devitalisierung von Geweben
20, 20', 20" Ablationssonde
30 Regel-/Steuereinrichtung
32 Messeinrichtung
34 Kühl-Leistungsregler
35 HF-Leistungsregler
40 Kältemittelquell
41 Ventil
42 Zulauf
50 HF-Generator
52 Leitung
P_{HF} HF-Leistung
P_{K} Kühlleistung
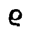 spezifischer Widerstand
Rᵢₛₜ Ist-Impedanz
Rₛₒₗₗ Soll-Impedanz
AP1 erster Arbeitspunkt
AP2 zweiter Arbeitspunkt

## Patentansprüche

1. Vorrichtung (10) zur Devitalisierung von biologischem Gewebe (300), umfassend:
- mindestens eine Ablationssonde (20, 20', 20"), die derart ausgebildete und angeordnete Kühleinrichtungen aufweist, dass durch deren Kühlleistung Gewebebereiche nahe der Ablationssonde (20, 20', 20") kühlbar sind, und die derart angeordnete und ausgebildete Elektrodeneinrichtungen aufweist, dass ein von einem HF-Generator (50) erzeugter HF-Behandlungsstrom in das Gewebe einleitbar ist,
- eine Regeleinrichtung (30), die derart ausgebildet und mit den Kühleinrichtungen sowie mit dem HF-Generator (50) verbunden ist, dass zu Beginn der Devitalisierung bei einer niedrigen, ersten Temperatur zur Vermeidung einer irreversiblen Veränderung des der Ablationssonde (20, 20', 20") unmittelbar benachbarten Gewebes und am Ende der Devitalisierung bei einer erhöhten, zweiten Temperatur gearbeitet wird,
**dadurch gekennzeichnet, dass**
die Regeleinrichtung (30) zum Einstellen der ersten Temperatur durch Vorgabe eines ersten Behandlungsstroms und zum Einstellen der zweiten Temperatur durch Vorgabe einer ersten Behandlungsspannung und jeweils einer entsprechenden Einstellung der Kühlleistung ausgebildet ist, wobei die Regeleinrichtung zur Feststellung der ersten und der zweiten Temperatur eine Impedanzmesseinrichtung (32) zur Messung einer Impedanz zwischen der Elektrodeneinrichtung und dem umgebenden Gewebe aufweist.

2. Vorrichtung (10) nach Anspruch 1,
**dadurch gekennzeichnet, dass**
die Regeleinrichtung (30) derart ausgebildet ist, dass die erste Temperatur so eingestellt wird, dass keine Vereisung des Gewebes auftritt.

3. Vorrichtung (10) nach einem der vorhergehenden Ansprüche,
**dadurch gekennzeichnet, dass**
die Regeleinrichtung (30) eine Konstantstromquelle zur Einstellung des ersten Behandlungsstroms umfasst.

4. Vorrichtung (10) nach einem der vorhergehenden Ansprüche,
**dadurch gekennzeichnet, dass**
die Regeleinrichtung eine Konstantspannungsquelle zur Einstellung der ersten Behandlungsspannung umfasst.

5. Vorrichtung (10) nach einem der vorhergehenden Ansprüche,
**dadurch gekennzeichnet, dass**
die Regeleinrichtung derart eingestellt ist, dass ein gleitender Übergang von einer Stromregelung zu einer Spannungsregelung durchgeführt wird.

6. Vorrichtung (10) nach einem der vorhergehenden Ansprüche, umfassend eine Vielzahl von Ablationssonden und Regeleinrichtungen,
**gekennzeichnet durch**
eine übergeordnete Steuer-/Regeleinrichtung (30), die derart ausgebildet ist, dass die jeweiligen Ablationssonden mit dazu gehörigen Regeleinrichtungen gemeinsam steuerbar sind.

## Claims

1. Device (10) for devitalizing biological tissue (300), comprising:
- at least one ablation probe (20, 20', 20"), which comprises cooling apparatuses embodied and arranged in such a way that, as a result of the cooling power thereof, tissue regions in the vicinity of the ablation probe (20, 20', 20") are coolable, and which comprises electrode apparatuses arranged and embodied in such a way that an RF treatment current generated by an RF generator (50) is introducible into the tissue,
- a regulation apparatus (30), which is embodied and connected to the cooling apparatuses and to the RF generator (50) in such a way that, at the start of the devitalization, work is carried out at a low, first temperature in order to avoid an irreversible change in the tissue directly adjacent to the ablation probe (20, 20', 20") and that, at the end of the devitalization, work is carried out at an increased, second temperature,
**characterized in that**
the regulation apparatus (30) is embodied to set the first temperature by prescribing a first treatment current and set the second temperature by prescribing a first treatment voltage and, in each case, set a corresponding setting of the cooling power, wherein, for the purposes of setting the first and the second temperature, the regulation apparatus comprises an impedance measurement apparatus (32) for measuring an impedance between the electrode apparatus and the surrounding tissue.

2. Device (10) according to Claim 1,
**characterized in that**
the regulation apparatus (30) is embodied in such a way that the first temperature is set so that no icing of the tissue occurs.

3. Device (10) according to one of the preceding claims,
**characterized in that**
the regulation apparatus (30) comprises a constant current source for setting the first treatment current.

4. Device (10) according to one of the preceding claims,
**characterized in that**
the regulation apparatus comprises a constant voltage source for setting the first treatment voltage.

5. Device (10) according to one of the preceding claims,
**characterized in that**
the regulation apparatus is set in such a way that a smooth transition is performed between current regulation and voltage regulation.

6. Device (10) according to one of the preceding claims, comprising a multiplicity of ablation probes and regulation apparatuses,
**characterized by**
a superordinate control/regulation apparatus (30), which is embodied in such a way that the respective ablation probes with the associated regulation apparatuses are controllable together.

## Revendications

1. Dispositif (10) de dévitalisation d'un tissu biologique (300), comprenant :
- au moins une sonde d'ablation (20, 20', 20") qui est configurée et qui présente des dispositifs de refroidissement disposés de telle sorte que leur puissance de refroidissement permet de refroidir les zones de tissu à proximité de la sonde d'ablation (20, 20', 20"), et laquelle présente des dispositifs à électrode disposés et configurés de telle sorte qu'un courant de manipulation HF généré par un générateur HF (50) peut être injecté dans le tissu,
- un dispositif de régulation (30) qui est configuré et relié avec les dispositifs de refroidissement ainsi qu'avec le générateur HF (50) de telle sorte qu'au début de la dévitalisation, le travail est effectué à une première température basse en vue d'éviter une modification irréversible du tissu directement voisin de la sonde d'ablation (20, 20', 20"), et à la fin de la dévitalisation à une deuxième température relevée,
**caractérisé en ce que**
le dispositif de régulation (30) est configuré pour régler la première température en prédéfinissant un premier courant de manipulation et pour régler la deuxième température en prédéfinissant une première tension de manipulation et à chaque fois un réglage correspondant de la puissance de refroidissement, le dispositif de régulation, en vue de déterminer la première et la deuxième température, présentant un dispositif de mesure d'impédance (32) servant à mesurer une impédance entre le dispositif à électrode et le tissu environnant.

2. Dispositif (10) selon la revendication 1,
**caractérisé en ce que**
le dispositif de régulation (30) est configuré de telle sorte que la première température est réglée de manière à ce qu'il ne se produise pas de givrage du tissu.

3. Dispositif (10) selon l'une des revendications précédentes,
**caractérisé en ce que**
le dispositif de régulation (30) comprend une source de courant constant destinée à régler le premier courant de manipulation.

4. Dispositif (10) selon l'une des revendications précédentes,
**caractérisé en ce que**
le dispositif de régulation (30) comprend une source de courant constant destinée à régler la première tension de manipulation.

5. Dispositif (10) selon l'une des revendications précédentes,
**caractérisé en ce que**
le dispositif de régulation est réglé de telle sorte qu'une transition glissante est effectuée d'une régulation du courant vers une régulation de la tension.

6. Dispositif (10) selon l'une des revendications précédentes, comprenant une pluralité de sondes d'ablation et de dispositifs de régulation,
**caractérisé par**
un dispositif de contrôle/régulation (30) superviseur qui est configuré de telle sorte que les sondes d'ablation respectives avec les dispositifs de régulation associés peuvent être commandés ensemble.
